# EUROPEAN PATENT APPLICATION

(11) **EP 4 295 784 A1**
(43) Date of publication of application: **27.12.2023**
(21) Application number: 22382593.6
(22) Date of filing: 22.06.2022
(51) Int. Cl.: A61B 17/06, A61B 17/00

(54) **SURGICAL ASSEMBLY, PROCESS FOR MANUFACTURING A SURGICAL ASSEMBLY AND DEVICE FOR MANUFACTURING A SURGICAL ASSEMBLY**

(71) Applicant: B. Braun Surgical, S.A., 08191 Rubi (Barcelona) (ES)
(72) Inventor: TURÓN DOLS, Pau, Rubí (ES); FUNK, Lutz, Rubí (ES); MOLINA, Alejandro José, Rubí (ES); PLANTÀ, Xavier, Cerdanyola del Vallès (ES); RIVILLAS, Francesc, Cerdanyola del Vallès (ES)
(74) Representative: Patentanwälte Ruff, Wilhelm, Beier, Dauster & Partner mbB

(57) **Abstract**

The invention refers to a surgical assembly (1), in particular in the form of a surgical needle-suture or surgical anchor-suture assembly, comprising
- a first element (10) having a proximal end portion (11) and a distal end portion (12) and
- a second element (20) having a proximal end portion (21) and a distal end portion (22),
wherein the distal end portion (12) of the first element (10) and the proximal end portion (21) of the second element (20) are joined to each other via a third element (30) covering the distal end portion (12) of the first element (10) and the proximal end portion (21) of the second element (20) and being attached to the distal end portion (12) of the first element (10) and proximal end portion (21) of the second element (20).

The invention further refers to a process and a device for manufacturing a surgical assembly.

## Description

The present invention relates to a surgical assembly, a process for manufacturing a surgical assembly and a device for manufacturing a surgical assembly.

### Background of the invention

The attachment end portion of a surgical needle may have four different configurations: a closed eye, French eye (split or spring), U-shaped channel or swaged (commonly known as eyeless).

Closed eye needles are very similar to common or domestic sewing needles, although the shape of the eye can be round, square or oblong. A disadvantage of closed eye needles is that the sutures have to be tied through the eye, thereby increasing the volume of suture that has to pass through the tissues. Further tying sutures to needles is a tedious and time-consuming process.

French eye needles are slitted from the inside of the eye to the distal end portion of the needle. Typically, the slit is provided with ridges to hold the suture in place. An advantage of French eye needles is that the suture can be easily inserted or removed from the eye, thereby simplifying suture attachment. However, it is possible during surgery for the needle to become detached from the suture and possibly lost within deep wounds.

Accordingly, French or closed eye needles are infrequently used in most surgical procedures, although they are still used for veterinary surgery with larger gauge sutures.

Virtually all surgical needles used in modern surgery are swaged or channel needles, where the suture is crimped to form a continuous unit although the security of suture attachment does vary with the suture size and the needle diameter. Swaged or channel needles have the great advantage that the suture is supplied with the needle attached in sterile packages so that immediate suturing of tissues is possible while greatly reducing the risk of contamination of the needle or suture. Most sutures are applied in combination swaged needles, which are considered more reliable in terms of bond strength and less traumatic to tissues.

During manufacture of swaged needles, a blind hole is drilled in the proximal end portion of the needle. For the manufacture of channel needles used in micro sutures, a U-shaped channel is cut at the proximal end portion of the needle and, after suture placement, the walls of the channel are closed around the suture to hold it firmly in place. Conventionally, the pull-out force to separate the suture from the needle is between 30% to 80% of the breaking load of the suture itself.

Recently, eyeless needless are also manufactured by laser, drilling a hole in the proximal end portion of the needle body, parallel to the axis of the needle. Compared to channel needles, eyeless needles have a smoother outer circumference while the length of the drilled hole is shorter than that required for the channel end. These are significant advantages, particularly the latter, as it allows the surgeon to hold the needle closer to the distal end portion.

Eyeless needles found rapid acceptance by surgeons because the difference in size between the needle and the suture, in other words, the ratio between the needle diameter and the suture diameter, is smaller than for the traditional channel needles. This reduction in the difference between needle and suture diameter facilitates surgical suturing and aids wound healing. The pull-out force to separate the suture from the needle is the same or slightly higher for the whole needle than for the channel crimp.

The significant advantages of the eyeless needle over the channel needle have resulted in the current market being largely dominated by sutures with eyeless needles, and the use of channel needles has been relegated to extremely small suture sizes, mainly for the assembly of sutures with thread diameters thinner than 50 µm.

Despite the remarkable progress made with respect to assemblies comprising surgical sutures and surgical needles or other surgical devices being adapted to introduce surgical sutures into biological tissues, it remains an ongoing need for further improved surgical assemblies.

### Object and solution

In view of the foregoing, the object underlying the present invention is therefore to make available a surgical assembly, a process for manufacturing a surgical assembly and a device for manufacturing a surgical assembly, in particular exhibiting further improved characteristics in comparison to the surgical needle-suture assemblies known from the prior art.

This object is accomplished by a surgical assembly according to the independent claim 1, a process for manufacturing a surgical assembly according to claim 12 and a device for manufacturing a surgical assembly according to claim 15. Preferred embodiments of the invention are defined in the dependent claims and the present description. The subject-matter and wording, respectively, of all claims is hereby incorporated into the description by explicit reference.

According to a first aspect, the invention relates to a surgical assembly, in particular in the form of a surgical device-suture assembly, preferably surgical needle-suture assembly or surgical anchor-suture assembly.

The assembly comprises the following:
- a first element having a proximal end portion and a distal end portion and
- a second element having a proximal end portion and a distal end portion.

The distal end portion of the first element and the proximal end portion of the second element are joined or connected to each other via a third element. More specifically, the distal end portion of the first element and the proximal end portion of the second element may be directly or indirectly joined or connected to each other via the third element.

The third element covers or coats, in particular only, the distal end portion of the first element and, in particular only, the proximal end portion of the second element. More specifically, the third element may completely or only partially, preferably completely, cover or coat the distal end portion of the first element and the proximal end portion of the second element.

Further, the third element may directly or indirectly, preferably directly, cover or coat the distal end portion of the first element and the proximal end portion of the second element.

Further, the third element is attached or connected, in particular bonded, to the distal end portion of the first element and proximal end portion of the second element. More specifically, the third element may be directly or indirectly, preferably directly, attached or connected, in particular bonded, to the distal end portion of the first element and the proximal end portion of the second element.

The present invention rests on the surprising finding that the ratio between the diameter of a surgical needle or another surgical device (such as a surgical anchor) being adapted to introduce a surgical suture into a biological, in particular human or non-human, tissue and the diameter of a surgical suture may be significantly reduced by a surgical assembly as defined according to the present invention. Thus, the risk of tissue trauma may be advantageously lowered, and consequently wound healing aided. In addition, an eye, a French eye, channel or hole at the proximal end portion of a surgical needle is dispensable, thereby simplifying the manufacturing process of the inventive surgical assembly and, in addition, increasing patient's safety.

The term "surgical assembly" as used according to the present invention may also be denoted as surgical combination, in particular surgical device-suture combination, preferably surgical needle-suture combination or surgical anchor-suture combination.

The term "proximal end portion" as used according to the present invention, in particular in terms of the first element and second element, refers to an end portion, in particular of the first element and second element, being arranged closer to a center or central axis of a body or organ of a user or practitioner, in particular physician or medical staff, preferably surgeon or surgical staff.

The term "distal end portion" as used according to the present invention, in particular in terms of the first element and second element, refers to an end portion, in particular of the first element and second element, being arranged more distant from a center or central axis of a body or organ of a user or practitioner, in particular physician or medical staff, preferably surgeon or surgical staff.

In an embodiment of the invention, the third element is cohesively, i.e. materially, attached or connected, in particular covalently or non-covalently bonded, particularly via Van der Waals force, hydrogen bonding, ionic interaction, coordinative bonding or a combination of at least two of the afore-said non-covalent bonding, to the distal end portion of the first element and/or proximal end portion of the second element. Preferably, the third element is cohesively attached or connected to the distal end portion of the first element and proximal end portion of the second element.

In a further embodiment of the invention, the third element is mechanically, in particular positively and/or frictionally, attached or connected to the distal end portion of the first element and/or proximal end portion of the second element. In particular, the third element may be mechanically attached or connected to the distal end portion of the first element and proximal end portion of the second element.

Further, the third element may be cohesively attached or connected to the distal end portion of the first element and mechanically attached or connected to the proximal end portion of the second element or vice versa. With respect of further feature in terms of the cohesive attachment/connection and mechanical attachment/connection, reference is made to the two preceding paragraphs. The features described therein in terms of the cohesive attachment/connection and mechanical attachment/connection do apply mutatis mutandis.

In a further embodiment of the invention, the third element is molded on the distal end portion of the first element and/or proximal end portion of the second element. Preferably, the third element is molded on the distal end portion of the first element and proximal end portion of the second element. More preferably, the third element is overmolded about the distal end portion of the first element and/or proximal end portion of the second element. Especially preferably, the third element is overmolded about the distal end portion of the first element and proximal end portion of the second element. Accordingly, it is especially preferred according to the present invention, that the third element is in the form of an overmolded component or overmolded piece. Advantageously, this embodiment of the invention may be realized at low costs and in a reliable and in particular customized fashion. Further, this embodiment of the invention results in an especially firm attachment or connection, in particular bonding, of the third element on the distal end portion of the first element and proximal end portion of the second element. Thus, the risk of detachment can be especially reduced.

In a further embodiment of the invention, the third element comprises or consists of a meltable, in particular injection-moldable, material, preferably polymer, more preferably synthetic polymer. Preferably, the polymer is selected from the group consisting of polyolefins, polyvinyl alcohols, polyamides, polyimides, polyesters, polyurethanes, in particular thermoplastic polyurethanes, polyhydroxyalkanoates, copolymers of at least two of the afore-said polymers and mixtures of at least two of the afore-said polymers.

In a further embodiment of the invention, the proximal end portion of the second element has a cross-section, in particular orthogonal or vertical cross-section, which is equal to a cross-section, in particular orthogonal or vertical cross-section, of the distal end portion of the first element.

In a further embodiment of the invention, the proximal end portion of the second element has a cross-section, in particular orthogonal or vertical cross-section, which is up to 30%, i.e. > 0% to 30%, larger than a cross-section, in particular orthogonal or vertical cross-section, of the distal end portion of the first element.

In a further embodiment of the invention, the distal end portion of the first element and the proximal end portion of the second element, in particular directly or indirectly, abut against each other. Preferably, the distal end portion of the first element and proximal end portion of the second element directly abut against each other, i.e. there is preferably no gap or space between the distal end portion of the first element and the proximal end portion of the second element.

In a further embodiment of the invention, the distal end portion of the first element and the proximal end portion of the second element are not directly joined to each other via the third element and the third element is additionally formed between the distal end portion of the first element and the proximal end portion of the second element.

In a further embodiment of the invention, the third element has a thickness, in particular layer or wall thickness, of 0.001 mm to 10 mm, in particular 0.005 mm to 5 mm, preferably 0.01 mm to 0.5 mm. The thickness may further be in the form of constant or varying thickness. For example, a portion of the third element covering or coating the distal end portion of the first element may have a thickness being different from a thickness of a portion of the third element covering or coating the proximal end portion of the second element and/or from a thickness of a portion of the third element being formed between the distal end portion of the first element and the proximal end portion of the second element. Alternatively, a portion of the third element covering or coating the proximal end portion of the second element may have a thickness being different from a thickness of a portion of the third element covering or coating the distal end portion of the first element and/or from a thickness of a portion of the third element being formed between the distal end portion of the first element and the proximal end portion of the second element. Alternatively, a portion of the third element covering or coating the distal end portion of the first element may have a thickness being equal to a thickness of a portion of the third element covering or coating the proximal end portion of the second element, in particular but being different from a thickness of a portion of the third element being formed between the distal end portion of the first element and proximal end portion of the second element. Alternatively, a portion of the third element covering or coating the distal end portion of the first element may have a thickness being equal to a thickness of a portion of the third element being formed between the distal end portion of the first element and proximal end portion of the second element, in particular but being different from a thickness of a portion of the third element covering or coating the proximal end portion of the second element. Alternatively, a portion of the third element covering or coating the proximal end portion of the second element may have a thickness being equal to a thickness of a portion of the third element being formed between the distal end portion of the first element and proximal end portion of the second element, in particular but being different from a thickness of a portion of the third element covering or coating the distal end portion of the first element. Alternatively, a portion of the third element covering or coating the distal end portion of the first element may have a thickness being equal to a thickness of a portion of the third element covering or coating the proximal end portion of the second element and being equal to a thickness of a portion of the third element being formed between the distal end portion of the first element and proximal end portion of the second element.

More specifically, when covering or coating the distal end portion of the first element, the third element may have a thickness of 0.001 mm to 10 mm, in particular 0.005 mm to 5 mm, preferably 0.01 mm to 0.5 mm. Further, when covering the proximal end portion of the second element, the third element may have a thickness of 0.001 mm to 10 mm, in particular 0.005 mm to 5 mm, preferably 0.01 mm to 0.5 mm. Furthermore, when being formed between the distal end portion of the first element and proximal end portion of the second element, the third element may have a thickness 0.001 mm to 10 mm, in particular 0.005 mm to 5 mm, preferably 0.01 mm to 0.5 mm.

In a further embodiment of the invention, the third element is in the form of a mantle, preferably sleeve, in particular tubular sleeve.

Further, the third element may have a length from 0.1 mm to 100 mm, in particular 0.5 mm to 50 mm, preferably 0.75 mm to 5 mm.

In a further embodiment of the invention, at least the distal end portion of the first element, in particular only the distal end portion of the first element or the entire first element, comprises or consists of a heat-labile or heat-sensitive, in particular thermoplastic, material. Alternatively, or in combination, at least the proximal end portion of the second element, in particular only the proximal end portion of the second element or the entire second element, may comprise or consist of a heat-labile or heat-sensitive material, in particular thermoplastic material.

The term "heat-labile material" and "heat-sensitive material", respectively, as used according to the present invention refers to a material which undergoes a change, in particular degradation, and/or becomes sticky, in particular due to melting, when being exposed to a defined elevated temperature.

Particularly, the heat-labile or heat-sensitive, in particular thermoplastic, material may have a degradation profile or degradation temperature of 30 °C to 400 °C, in particular 36 °C to 250 °C.

Further, the heat-labile or heat-sensitive, in particular thermoplastic, material is preferably selected from the group consisting of polyolefins, polyvinyl alcohols, polyamides, polyimides, polyesters, polyurethanes, in particular thermoplastic polyurethanes, polyhydroxyalkanoates, copolymers of at least two of the afore-said polymers, in particular synthetic polymers, and mixtures of at least two of the afore-said polymers, in particular synthetic polymers.

In a further embodiment of the invention, at least the proximal end portion of the second element, in particular only the proximal end portion of the second element or the entire second element, comprises or consists of a metallic material. Alternatively, or in combination, at least the distal end portion of the first element, in particular only the distal end portion of the first element or the entire first element, may comprise or consist of a metallic material.

The term "metallic material" as used according to the present invention refers to any material which is characterized by metallic bonding and/or constituted by metallic ions.

Preferably, the metallic material is selected from the group consisting of steel, stainless steel, nickel-titanium (NiTi) alloy, cobalt-chromium (CoCr) alloy, tungsten-rhenium (W-Re) alloy, and mixtures, in particular alloys, of at least two of the afore-said metallic materials.

Preferably, the first element and/or second element, in particular first element and second element, have/has an elongate form or are/is in an elongate form.

Further, the first element or at least the distal end portion of the first element may have a corner-free, i.e. round, cross-section. For example, the first element or at least the distal end portion of the first element may have a circular, oval or elliptical cross-section. Alternatively, the first element or at least the distal end portion of the first element may have a polygonal, in particular triangular, oblong, square, pentagonal, hexagonal, heptagonal, octagonal, nonagonal, decagonal or star-like, cross-section.

Further, the second element or at least the proximal end portion of the second element may have a corner-free, i.e. round, cross-section. For example, the second element or at least the proximal end portion of the second element may have a circular, oval or elliptical cross-section. Alternatively, the second element or at least the proximal end portion of the second element may have a polygonal, in particular triangular, oblong, square, pentagonal, hexagonal, heptagonal, octagonal, nonagonal, decagonal or star-like, cross-section.

In a further embodiment of the invention, the first element is in the form of a surgical suture and the second element is in the form of a surgical device being adapted to introduce the surgical suture into or through a biological, in particular human or non-human, tissue. The surgical suture may be in the form of a monofilament, pseudo monofilament or multifilament, in particular braided or twisted multifilament. Further, the surgical suture may have a diameter of 0.015 mm to 2 mm, in particular 0.03 mm to 1.2 mm, preferably 0.04 mm to 1 mm. Preferably, the surgical device being adapted to introduce the surgical suture into or through a biological, in particular human or non-human, tissue is in the form of a surgical needle or surgical anchor. The term "surgical anchor" as used according to the present invention refers to any part that is able to retain a biological tissue. The surgical needle may, for example, have a straight shape, 1/4 circle shape, 3/8 circle shape, 1/2 circle shape, 5/8 circle shape, a compound curve, a half curved shape (also known as ski needle) or being half curved at both ends of a straight segment (also known as canoe needle). Further, the surgical needle may have a taper point (needle body is round and tapers smoothly to a point), cutting point (needle body is triangular and has a sharpened cutting edge on the inside curve), a reverse cutting point (cutting edge on the outside), a trocar point or taper cut point (needle body is round and tapered, but ends in a small triangular cutting point), blunt points (for sewing friable tissues) or a side cutting or spatula point (flat on top and bottom with a cutting edge along the front to one side). Further, the needle may be in the form of an atraumatic needle. Principally, the surgical needle may have a closed eye or a French eye at its proximal end portion. Alternatively, the needle may be principally in the form of an eyeless needle, in particular swaged or channel needle. More preferably, however, the surgical needle, in particular a proximal end portion of the surgical needle, is free of any attachment element for attaching the surgical suture, in particular an eye, a channel, or hole. Further, the surgical device being adapted to introduce the surgical suture into or through a biological, in particular human or non-human, tissue, in particular the surgical needle or surgical anchor, may have a diameter of 0.02 mm to 2 mm, in particular 0.03 mm to 1.8 mm, preferably 0.05 mm to 1.68 mm. Furthermore, the surgical assembly may have a ratio of the diameter of the surgical suture to the diameter of the surgical device being adapted to introduce the surgical suture into or through a biological, in particular human or non-human, tissue, in particular to the diameter of the surgical needle or surgical anchor, of 0.5 to 2, in particular 0.75 to 1.5, preferably 0.9 to 1.1.

Especially preferably, the first element is in the form of a surgical suture and the second element is in the form of a surgical needle. With respect to further features and advantages of the surgical suture and the surgical needle, reference is made in its entirety particularly to the previous paragraph. The advantages and features described therein with respect to the surgical suture and the surgical needle do apply, mutatis mutandis.

According to a second aspect, the present invention refers to a process for manufacturing a surgical assembly, in particular according to the first aspect of the invention.

The process comprises the step of:
a) forming, in particular directly or indirectly forming, a third element on a distal end portion, in particular only on a distal end portion, of a first element and on a proximal end portion, in particular only on a proximal end portion, of a second element such that the distal end portion of the first element and the proximal end portion of the second element are, in particular directly or indirectly, joined or connected to each other via the third element and the third element covers or coats, in particular directly or indirectly covers or coats, the distal end portion of the first element and the proximal end portion of the second element and is, in particular directly or indirectly, attached or connected, in particular bonded, to the distal end portion of the first element and proximal end portion of the second element.

Preferably, the distal end portion of the first element and/or proximal end portion of the second element, in particular distal end portion of the first element and proximal end portion of the second element, are/is subjected to a surface treatment, in particular mechanical surface treatment and/or chemical surface treatment. Thus, the strength of the third element's attachment or connection to the distal end portion of the first element and/or proximal end portion of the second element may be advantageously increased. The mechanical surface treatment may be selected from the group consisting of abrasive blasting, sand blasting, burnishing, grinding, honing, mass finishing, industrial etching, laser ablation, laser engraving and combinations of at least two of the afore-said mechanical surface treatments. The chemical surface treatment may be selected from the group consisting of chemical vapor deposition (CVD), physical vapor deposition (PVD), plasma spraying, powder coating, thin-film deposition, electroplating, electrophoretic deposition, galvanizing, mechanical plating, sputter deposition, passivation, plasma activation, flame activation and combinations of at least two of the afore-said chemical surface treatments.

Further, the distal end portion of the first element and the proximal end portion of the second element are preferably abutted against each other before or during step a). Thus, an especially firm conjunction or connection between the distal end portion of the first element and the proximal end portion of the second element via the third element may be accomplished.

Alternatively, the distal end portion of the first element and the proximal end portion of the second element are approximated up to a defined distance, in particular to a distance of 5 mm to 10 mm, preferably 1 mm to 2 mm, before or during step a).

In a further embodiment of the invention, step a) is carried out by applying a molten material on the distal end portion of the first element and proximal end portion of the second element and subsequently cooling the molten material applied on the distal end portion of the first element and proximal end portion of the second element to solidify to the third element. Preferably, the molten material is provided by heating a meltable material at a temperature of 100 °C to 350 °C, in particular 120 °C to 300 °C, preferably 200 °C to 260 °C. Further, the meltable material may be heated for a time period of 0.1 s to 20 s, in particular 0.5 s to 10 s, preferably 0.75 s to 5 s. Further, after having been applied on the distal end portion of the first element and proximal end portion of the second element, the molten material is preferably cooled at a temperature of 10 °C to 60 °C, in particular 15 °C to 40 °C, preferably 20 °C to 30 °C. Further, the molten material may be cooled for a time period of 0.5 s to 600 s, in particular 0.75 s to 300 s, preferably 3 s to 30 s.

In a further embodiment of the invention, a molten material is applied transversally, i.e. inclined or sloped (under an acute angle, i.e. an angle between 0° and 90°), with respect to a longitudinal axis of the first element on the distal end portion of the first element, in particular such that the distal end portion of the first element is drawn or dragged in the direction of the proximal end portion of the second element, preferably such that either the distal end portion of the first element and the proximal end portion of the second element, in particular directly or indirectly, abut against each other or are spaced apart from each other at a defined distance, in particular at a distance of 0.05 mm to 5 mm, preferably 1 mm to 2 mm. With respect to further features and advantages regarding processing of the meltable material, reference is made to the previous paragraph. The features and advantages described in the previous paragraph do also apply, mutatis mutandis, with respect to the embodiment described in this paragraph.

In a further embodiment of the invention, step a) is carried out by injection overmolding, i.e. by injection molding the third element over or about the distal end portion of the first element and proximal end portion of the second element.

Preferably, step a) is carried out by ultrasonic injection overmolding, i.e. ultrasonic injection molding the third element over or about the distal end portion of the first element and proximal end portion of the second element. For example, an ultrasonic frequency of 20 kHz to 60 kHz, in particular 25 kHz to 35 kHz, preferably 29 kHz to 31 kHz, may be applied, in particular for a time period of 0,1 s to 30 s, in particular 1 s to 10 s, preferably 4 s to 6 s. By using ultrasonic injection overmolding, a meltable material used for forming the third element is heated up to its melting point by applying ultrasonic energy.

With respect to further features and advantages of the process, in particular in terms of the first element, second element and third element, reference is made in its entirety to the embodiments described under the first aspect of the present invention. The features and advantages described under the first aspect of the present invention do also apply, mutatis mutandis, with respect to the process according to the second aspect of the present invention.

According to a third aspect, the present invention refers to a device for manufacturing a surgical assembly, in particular according to the first aspect of the present invention.

The device comprises a mold comprising
- a first mold cavity being adapted to receive a first element, wherein the first element has a proximal end portion and a distal end portion,
- a second mold cavity being adapted to receive a second element, wherein the second element has a proximal end portion and a distal end portion,
- at least one positioning element being adapted to position the distal end portion of the first element inside the first mold cavity,
- at least one positioning element being adapted to position the proximal end portion of the second element inside the second mold cavity and
- at least one inlet channel being adapted to guide a molten material into the first mold cavity and/or second mold cavity, in particular to form a third element on the distal end portion of the first element and proximal end portion of the second element.

The term "at least one positioning element" as used according to the present invention may mean only one positioning element or a plurality of positioning elements, i.e. two or more positioning elements.

Further, the term "at least one inlet channel" as used according to the present invention may mean only one inlet channel or a plurality of inlet channels, i.e. two or more inlet channels.

Preferably, the first mold cavity and the second mold cavity are fluidly connected to each other.

Further, the first mold cavity preferably has a cross-section which is larger than a cross-section of the first element or distal end portion of the first element.

Further, the first mold cavity is preferably adapted to receive the first element such that a first tubular gap or space is defined between an inner surface or contour of the first mold cavity and an outer surface or contour of the first element or distal end portion of the first element.

The first tubular gap may have a thickness, in particular constant or varying thickness, from 0.001 mm to 10 mm, in particular 0.005 mm to 5 mm, preferably 0.01 mm to 0.5 mm. Further, the first tubular gap may have a length from 0.1 mm to 50 mm, in particular 0.5 mm to 50 mm, preferably 0.75 mm to 2.5 mm.

Further, the second mold cavity preferably has a cross-section which is larger than a cross-section of the second element or proximal end portion of the second element.

Further, the second mold cavity is preferably adapted to receive the second element such that a second tubular gap is defined between an inner surface or contour of the second mold cavity and an outer surface or contour of the second element or proximal end portion of the second element.

Further, the second tubular gap may have a thickness, in particular constant or varying thickness, 0.001 mm to 10 mm, in particular 0.005 mm to 5 mm, preferably 0.01 mm to 0.5 mm. Further, the second tubular gap may have a length from 0.1 mm to 50 mm, in particular 0.5 mm to 50 mm, preferably 0.75 mm to 2.5 mm.

Further, the mold is preferably separable or decouplable into two mold components, wherein the mold components, when being pieced together or coupled, form the first mold cavity and the second mold cavity.

Further, the at least one inlet channel is preferably part of the first mold component and in particular runs transversally, i.e. inclined or sloped (under an acute angle, i.e. an angle between 0° and 90°), with respect to a longitudinal axis of the first mold component or first mold cavity through a wall of the first mold component.

Further, the at least one positioning element being adapted to position the distal end portion of the first element inside the first mold cavity preferably extends, in particular inwardly, from an inner surface or contour of the first mold cavity.

Preferably, the at least one positioning element being adapted to position the distal end portion of the first element inside the first mold cavity is in the form of two positioning elements, in particular extending, preferably inwardly, from opposite sides of an inner surface or contour of the first mold cavity.

More preferably, the at least one further positioning element being adapted to receive the distal end portion of the first element inside the first mold cavity is in the form of at least one lug, i.e. only one lug or a plurality of lugs, i.e. two or more lugs. Especially preferably, the at least one positioning element being adapted to receive the distal end portion of the first element inside the first mold cavity is in the form of two lugs, in particular extending, preferably inwardly, from opposite sides of an inner surface or contour of the first mold cavity.

Further, the at least one positioning element being adapted to position the proximal end portion of the second element inside the second mold cavity preferably extends, in particular inwardly, from an inner surface or contour of the second mold cavity.

Preferably, the at least one positioning element being adapted to position the proximal end portion of the second element inside the second mold cavity is in the form of two positioning elements, in particular extending, preferably inwardly, from opposite sides of an inner surface or contour of the second mold cavity.

More preferably, the at least one further positioning element being adapted to receive the proximal end portion of the second element inside the second mold cavity is in the form of at least one lug, i.e. only one lug or a plurality of lugs, i.e. two or more lugs. Especially preferably, the at least one positioning element being adapted to receive the proximal end portion of the second element inside the second mold cavity is in the form of two lugs, in particular extending, preferably inwardly, from opposite sides of an inner surface or contour of the second mold cavity.

Further, the device may further comprise a guiding element being adapted to guide the first element inside the first mold cavity. The guiding element may in particular comprise or be in the form of a channel or elongate recess. Further, the guiding element may be in the form of an insert comprising a guiding channel or an elongate recess. The channel or elongate recess may have a length of 1 mm to 150 mm, in particular 3 mm to 50 mm, preferably 5 mm to 30 mm. Thus, correct positioning of the first element inside the first mold cavity may be advantageously facilitated.

With respect to further features and advantages of the device, in particular in terms of the first element, second element and third element, reference is made in its entirety to the previous description, in particular the embodiments described under the first aspect of the present invention. The features and advantages described in the previous description, in particular under the first aspect of the present invention, in particular in terms of the first element, second element and third element, do also apply, mutatis mutandis, with respect to the device according to the third aspect of the present invention.

Further features and advantages of the invention will become clear from the following figures, descriptions thereof and examples in combination with the subject-matter of the dependent claims. The individual features can be realized either singularly or severally in combination in one embodiment of the invention. The preferred embodiments merely serve for illustration and better understanding of the invention and are not to be understood as in any way limiting the invention.

### BRIEF DESCRIPTOPN OF THE FIGURES

In the figures, the following is schematically displayed:
- Fig. 1:: an embodiment of a surgical assembly according to the invention,
- Fig. 2a:: a longitudinal section view of an embodiment of a device according to the Invention and
- Fig. 2b:: an explosive view of a device as shown in Fig. 2a.

### DETAILLED FIGURE DESCRIPTION

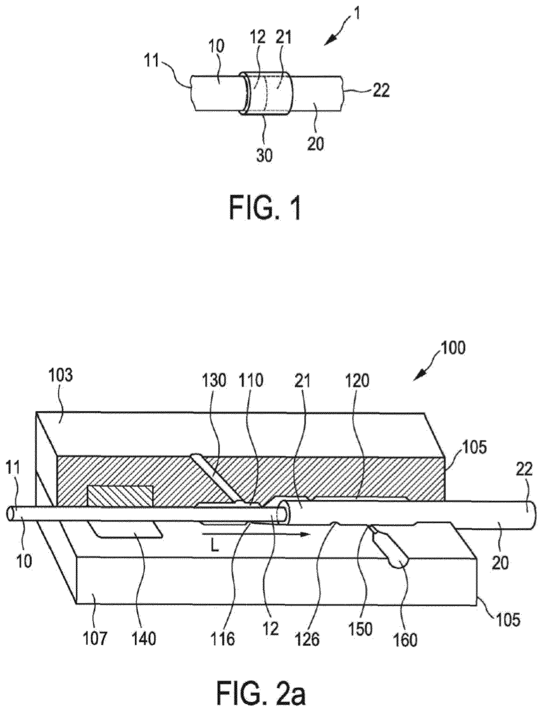

Fig. 1 schematically shows a surgical assembly 1 according to the present invention.

The surgical assembly 1 comprises a first element 10 having a proximal end portion 11 and a distal end portion 12 and a second element 20 having a proximal end portion 21 and a distal end portion 22.

Further, the surgical assembly 1 comprises a third element 30. The distal end portion 12 of the first element 10 and the proximal end portion 21 of the second element 20 are joined to each other via the third element 30.

In particular, the proximal end portion 21 of the second element 20 has a cross-section, in particular orthogonal or vertical cross-section, which is up to 30%, larger than a cross-section, in particular orthogonal or vertical cross-section, of the distal end portion 12 of the first element 10.

The third element 30, in particular completely, covers the distal end portion 12 of the first element 10 and the proximal end portion 21 of the second element 20.

Further, the third element 30 is attached, in particular bonded, to the distal end portion 12 of the first element 10 and the proximal end portion 21 of the second element 20. For example, the third element 30 may be mechanically or chemically, in particular covalently or non-covalently, in particular via Van der Waals force, attached, in particular bonded, to the distal end portion 12 of the first element 10 and the proximal end portion 21 of the second element 20.

Preferably, the distal end portion 12 of the first element 10 and the proximal end portion 21 of the second element 20 directly abut against each other.

Alternatively, the distal end portion 12 of the first element 10 and the proximal end portion 21 of the second element 20 may be spaced apart from each other. In this case, the third element 30 is preferably also formed between the distal end portion 12 and the proximal end portion 21.

Further, the third element 30 is preferably overmolded about the distal end portion 12 of the first element 10 and the proximal end portion 21 of the second element 20. In other words, the third element 30 is preferably in the form of an overmolding component or overmolding part.

Preferably, the third element 30 comprises or consists of a meltable, in particular injection-moldable, material. Preferably, the material is selected from the group consisting of polyolefins, polyvinyl alcohols, polyamides, polyimides, polyesters, polyurethanes, in particular thermoplastic polyurethanes, polyhydroxyalkanoates, copolymers of at least two of the afore-said polymers, in particular synthetic polymers, and mixtures of at least two of the afore-said polymers, in particular synthetic polymers.

Further, the third element 30 may have a thickness from 0.001 mm to 10 mm, in particular 0.005 mm to 5 mm, preferably 0.01 mm to 0.5 mm. Further, the third element 30 may have a length of 0.1 mm to 100 mm, in particular 0.5 mm to 50 mm, preferably 0.75 mm to 5 mm.

Further, the third element 30 is preferably in the form of a mantle, in particular sleeve, preferably tubular sleeve.

Further, at least the distal end portion 12 of the first element 10, in particular only the distal end portion 12 of the first element 10 or the entire first element 10, preferably comprises or consists of a heat-labile, in particular thermoplastic, material. The material may have a degradation temperature or profile of 30 °C to 240 °C. Furthermore, the material may be selected from the group consisting of polyolefins, polyvinyl alcohols, polyamides, polyimides, polyesters, polyurethanes, in particular thermoplastic polyurethanes, polyhydroxyalkanoates, copolymersof at least two of the afore-said polymers, in particular synthetic polymers, and mixtures of at least two of the afore-said polymers, in particular synthetic polymers.

Further, the first element 10 is preferably in the form of a surgical suture. The surgical suture may be in the form of a monofilament, pseudo monofilament or multifilament such as a twisted or braided multifilament.

Further, at least the proximal end portion 21 of the second element 20, in particular only the proximal end portion 21 of the second element 20 or the entire second element 20, preferably comprises or consists of a metallic material, in particular selected from the group consisting of steel, stainless steel, nickel-titanium (NiTi) alloy, cobalt-chromium (CoCr) alloy, tungsten-rhenium (W-Re) alloy and mixtures, in particular alloys, of at least two of the afore-said metallic materials.

Preferably, the second element 20 is in the form of a surgical needle or surgical anchor.

More preferably, the surgical assembly 1 is in the form of a surgical needle-suture assembly or combination or in the form of a surgical anchor-suture assembly or combination.

The surgical assembly 1 advantageously facilitates reduction of the ratio between the diameter of the second element, in particular surgical needle or surgical anchor, 20 and the diameter of the first element, in particular surgical suture, 10 as well as improvement of the reliability of conjunction or connection between the distal end portion 12 of the first element 10 and the proximal end portion 21 of the second element 20 via the third element 30.

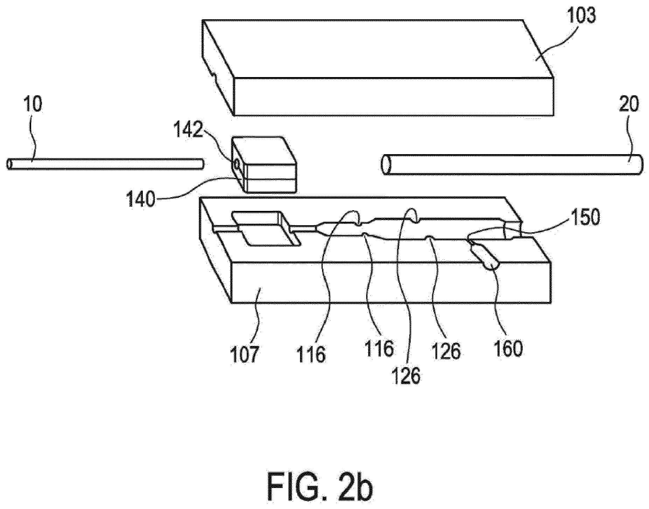

Figs. 2a and 2b schematically show a device 100 for manufacturing a surgical assembly according to the present invention, in particular by means of injection overmolding, preferably ultrasonic injection overmolding.

The device 100 comprises a mold 105. The mold 105 comprises a first mold cavity 110 being adapted to receive a first element 10 having a proximal end portion 11 and a distal end portion 12. Further, the mold 105 comprises a second mold cavity 120 being adapted to receive a second element 20 having a proximal end portion 21 and a distal end portion 22. Further, the mold 105 comprises at least one positioning element 116 being adapted to position the distal end portion 12 of the first element 10 inside the first mold cavity 110. Further, the mold 105 comprises at least one positioning element 126 being adapted to position the proximal end portion 21 of the second element 20 inside the second mold cavity 120. Further, the mold 105 comprises at least one inlet channel 130 being adapted to guide a molten material into the first mold cavity 110.

The mold 105 is preferably separable or decouplable into a first mold component 103 and a second mold component 107. The first mold component 103 may also be termed as ejection cavity plate, while the second mold component 107 may also be termed as injection cavity plate, according to the present invention. Preferably, when being pieced together or coupled, the first mold component 103 and the second mold component 107 form the first mold cavity 110 and the second mold cavity 120. Expediently, the first mold cavity 110 and the second mold cavity 120 are fluidly connected to each other.

Further, the first mold cavity 110 has a cross-section being larger than a cross-section of the first element 10 or distal end portion 12 of the first element 10, in particular such that a first tubular gap is defined between an inner surface of the first mold cavity 110 and an outer surface of the first element 10 or distal end portion 12 of the first element 10.

Further, the second mold cavity 120 has a cross-section being larger than a cross-section of the second element 20 or proximal end portion 21 of the second element 20, in particular such that a second tubular gap is defined between an inner surface of the second mold cavity 120 and an outer surface of the second element 20 or proximal end portion 21 of the second element 20.

Further, the at least one positioning element 116 being adapted to position the distal end portion 12 of the first element 10 inside the first mold cavity 110 is in the form of two lugs extending inwardly and oppositely from an inner surface of the first mold cavity 110. Further, the at least one positioning element 126 being adapted to position the proximal end portion 21 of the second element 20 inside the second mold cavity 120 is in the form of two lugs extending inwardly and oppositely from an inner surface of the second mold cavity 120. Thus, both the distal end portion 12 and the proximal end portion 21 may be advantageously held in position within the first mold cavity 110 and second mold cavity 120.

The at least one inlet channel 130 is preferably part of the first mold component 103. Further, the at least one inlet channel 130 preferably runs transversally, i.e. under an acute angle (angle between 0° and 90°), with respect to a longitudinal axis L of the first mold cavity 110 through a wall of the first mold component 103. Thus, a molten material being used for forming, in particular overmolding, a third element around the distal end potion 12 of the first element 10 and the proximal end portion 21 of the second element 20 may be guided into the first mold cavity 110 such that the molten material flows towards (in the direction of) the second mold cavity 120 and thereby exerts a dragging force on the outer surface of the distal end portion 12 of the first element 10. This aids that the distal end portion 12 of the first element 10 and the proximal end portion 21 of the second element 20 are either directly abutted against each other or are approximated to a defined distance, for example, to a distance of 0.5 mm to 2 mm, during their conjunction or connection via the third element 30.

Further, the device 100 may also comprise a guiding element 140 being adapted to guide the first element 10, in particular its distal end portion 12, inside the first mold cavity 110. The guiding element 140 may be in particular in the form of an insert having a guiding channel 142.

Further, the device 100, in particular the first mold component 103 and/or second mold component 107, may comprise a runner 150 and a gate 160.

Preferably, the runner 150 is in the form of a channel. The runner 150 may be directly machined into the first mold component 103 and/or second mold component 107. Further, the runner 150 is preferably adapted to direct the flow of the molten material towards the first mold cavity 110 and/or second mold cavity 120.

The gate 160 is preferably in the form of an opening, in particular small opening, in the mold 105 through which the molten material being supplied by the runner 150 fills the first mold cavity 110 and/or second mold cavity 120. In particular, the gate and opening, respectively 160 may be in the form of a drilled circular hole or may have a rectangular or oblong shape, in particular may be milled to produce a rectangular or oblong shape.

With respect to further features and advantages of the embodiments shown in Figs. 1, 2a and 2b, reference is made in its entirety to the general description.

### EXAMPLE SECTION

A first element consisting of a straight cylindrical needle made of AISI 302 stainless steel was attached to a second element consisting of a surgical thread made of 95% polypropylene and 5% polyethylene by means of a third element consisting of an overmolded sleeve made of pure polypropylene.

The surface of the needle was fine polished to a Ra of 0.4 and plasma activated during 4 s prior to its placing in a steel mold. The needle and surgical thread were butt-positioned against each other with no gaps between them. Both elements were held in position during the injection process by a set of hardened steel lugs.

The third element was overmolded by ultrasonic injection with the following process parameters:
Mold temperature: 75 °C
Injection chamber temperature: 260 °C
Injection speed: 16 mm/s
Sonotrode frequenzy: 29 kHz
Sonotrode time: 4s
Compaction force: 14000 N
Cooling time: 5 s

After the ultrasonic injection molding step, the sprue was precisely cut off from the resulting assembly by means of a small tailor-made guillotine-like device with a 0.2 mm thick AISI 420 steel blade. The resulting surgical assembly did not require any additional finishing steps such as deflashing or polishing of the part line.

## Claims

1. A surgical assembly (1), in particular in the form of a surgical needle-suture or surgical anchor-suture assembly, comprising
- a first element (10) having a proximal end portion (11) and a distal end portion (12) and
- a second element (20) having a proximal end portion (21) and a distal end portion (22),
wherein the distal end portion (12) of the first element (10) and the proximal end portion (21) of the second element (20) are joined to each other via a third element (30) covering the distal end portion (12) of the first element (10) and the proximal end portion (21) of the second element (20) and being attached to the distal end portion (12) of the first element (10) and proximal end portion (21) of the second element (20).

2. The surgical assembly (1) according to claim 1, wherein the third element (30) is cohesively or mechanically attached to the distal end portion (12) of the first element (10) and proximal end portion (21) of the second element (20).

3. The surgical assembly (1) according to claim 1 or 2, wherein the third element (30) is molded on, in particular overmolded about, the distal end portion (12) of the first element (10) and proximal end portion (21) of the second element (20).

4. The surgical assembly (1) according to any of the preceding claims, wherein the third element (30) comprises or consists of a meltable material, in particular injection-moldable material, preferably selected from the group consisting of polyolefins, polyvinyl alcohols, polyamides, polyimides, polyesters, polyurethanes, in particular thermoplastic polyurethanes, polyhydroxyalkanoates, copolymers of at least two of the afore-said polymers, in particular synthetic polymers, and mixtures of at least two of the afore-said polymers, in particular synthetic polymers.

5. The surgical assembly (1) according to any of the preceding claims, wherein the proximal end portion (21) of the second element (20) has a cross-section, in particular orthogonal cross-section, which is equal or up to 30 % larger than a cross-section, in particular orthogonal cross-section, of the distal end portion (12) of the first element (10).

6. The surgical assembly (1) according to any of the preceding claims, wherein the distal end portion (12) of the first element (10) and the proximal end portion (21) of the second element (20) directly abut against each other.

7. The surgical assembly (1) according to any of the claims 1 to 5, wherein the distal end portion (12) of the first element (10) and the proximal end portion (21) of the second element (20) are not directly joined to each other via the third element (30) and the third element (30) is additionally formed between the distal end portion (12) of the first element (10) and the proximal end portion (21) of the second element (20).

8. The surgical assembly (1) according to any of the preceding claims, wherein the third element (30) is in the form of a mantle, preferably sleeve.

9. The surgical assembly (1) according to any of the preceding claims, wherein at least the distal end portion (12) of the first element (10) comprises or consists of a heat labile material, in particular having a degradation profile of 30 °C to 400 °C, and/or being selected from the group consisting of polyolefins, polyvinyl alcohols, polyamides, polyimides, polyesters, polyurethanes, in particular thermoplastic polyurethanes, polyhydroxyalkanoates, copolymers of at least two of the afore-said polymers, in particular synthetic polymers, and mixtures of at least two of the afore-said polymers, in particular synthetic polymers.

10. The surgical assembly (1) according to any of the preceding claims, wherein at least the proximal end portion (21) of the second element (20) comprises or consists of a metallic material, in particular selected from the group consisting of stainless steel, nickel-titanium (NiTi) alloy, cobalt-chromium (CoCr), alloy, tungsten-rhenium (W-Re) alloy and mixtures of at least two of the afore-said metallic materials.

11. The surgical assembly (1) according to any of the preceding claims, wherein the first element (10) is in the form of a surgical suture and the second element is in the form of a surgical needle or surgical anchor.

12. A process for manufacturing a surgical assembly (1) according to any of the preceding claims, comprising the step of
a) forming a third element (30) on a distal end portion (12) of a first element (10) and a proximal end portion (21) of a second element (20) such that the distal end portion (12) of the first element (10) and the proximal end portion (21) of the second element (20) is joined to each other via the third element (30) and the third element (30) covers the distal end portion (12) of the first element (10) and the proximal end portion (21) of the second element (20) and is attached to the distal end portion (12) of the first element (10) and the proximal end portion (21) of the second element (20).

13. The process according to claim 12, wherein step a) is carried out by applying a molten material transversally with respect to a longitudinal axis L of the first element (10) on the distal end portion (12) of the first element (10) such that the distal end portion (12) of the first element (10) is drawn in the direction of the second element (20), in particular such that either the distal end portion (12) of the first element (10) and the proximal end portion (21) of the second element (20) abut against each other or a spaced apart from each other at a defined distance.

14. The process according to claim 12 or 13, wherein step a) is carried out by injection overmolding, in particular ultrasonic injection overmolding.

15. A device (100) for manufacturing a surgical assembly (1) according to any of the claims 1 to 11, comprising a mold (105) comprising
- a first mold cavity (110) being adapted to receive a first element (10) having a proximal end portion (11) and a distal end portion (12) and
- a second mold cavity (120) being adapted to receive a second element (20) having a proximal end portion (21) and a distal end portion (22),
- at least one positioning element (116) being adapted to position the distal end portion (12) of the first element (10) inside the first mold cavity (110),
- at least one positioning element (126) being adapted to position the proximal end portion (21) of the second element (20) inside the second mold cavity (120) and
- at least one inlet channel (130) being adapted to guide a molten material into the first mold cavity (110) and/or second mold cavity (120) to form a third element (30) on the distal end portion (12) of the first element (10) and proximal end portion (21) of the second element (20).
